# EUROPEAN PATENT APPLICATION

(11) **EP 2 786 724 A1**
(43) Date of publication of application: **08.10.2014**
(21) Application number: 14172127.4
(22) Date of filing: 30.03.2012
(51) Int. Cl.: A61F 2/00, A61B 17/00, A61B 18/14

(54) **Radio frequency-based surgical implant fixation apparatus**

(30) Priority: 31.03.2011 US 201161469898 P; 07.02.2012 US 201213367529
(62) Divisional of application: 12162605.5
(71) Applicant: Covidien LP, Mansfield, MA 02048 (US)
(72) Inventor: Rick, Kyle, Boulder, CO Colorado 80303 (US); Valentine, Christopher A., Boulder, CO Colorado 80304 (US); Alexander, Scott, Westminister, CO Colorado 80020 (US); Johnson, Kristin D., Louisville, CO Colorado 80027 (US)
(74) Representative: Morgan, Marc

(57) **Abstract**

A radio frequency-based surgical implant fixation apparatus, comprising: a housing having a shaft operably coupled thereto and defining a longitudinal axis therethrough, the housing adapted to connect to a source of electrosurgical energy; and an approximator assembly having an elongated rod coaxially coupled to the shaft and configured to reciprocate therethrough from a retracted position to an extended position, the elongated rod including a plurality of delivery arms operably coupled to a distal end thereof, the plurality of delivery arms configured to releasably receive a portion of a surgical implant and connect to the energy source to selectively transmit electrosurgical energy to the portion of the surgical implant to fuse the portion of the surgical implant to tissue upon actuation thereof, wherein at least one aperture is defined in the plurality of delivery arms, the at least one aperture being in fluid communication with a suction source configured to evacuate air from between the surgical implant and tissue to facilitate positioning the surgical implant thereagainst in a substantially taut configuration.

## Description

### CROSS REFERENCE TO RELATED APPLICATION

The present application claims the benefit of and priority to U.S. Provisional Application Serial No.61/469,898, filed on March 31, 2011, the entire contents of which are incorporated herein by reference.

### BACKGROUND

### Technical Field

The present disclosure relates to a radio frequency (RF) based surgical implant fixation apparatus and, more particularly, to a radio frequency-based surgical implant fixation apparatus including an approximator assembly having a plurality of electrically conductive delivery arms for positioning a surgical implant adjacent to tissue for subsequent fusion thereto. Fusion enhancements and non-RF methods of adhering the implant to tissue are also disclosed.

### Description of the Related Art

Hernias are abnormal protrusions of an organ or other body structure through a defect or natural opening in a covering membrane, e.g., a wall of a cavity that normally contains the organ or other body structure. For example, inguinal hernias are, typically, caused by soft tissue from the intestines protruding through the inguinal wall. Ventral hernias, on the other hand, are caused by internal organs pushing through to a weak spot in the abdominal wall.

The use of prosthetic mesh has now become accepted practice in the treatment of patients with both inguinal and ventral hernias, as well as other types of hernias, e.g., hiatal, femoral, umbilical, diaphragmatic, etc. To endoscopically apply the mesh for hernia repair, a surgical region (i.e., adjacent the cavity wall) is, typically, insufflated. Subsequently, a surgeon selects points on the cavity wall where the surgeon believes a peripheral edge of the mesh, i.e., the expected corners of a mesh (assuming a rectangular mesh), will be affixed. In certain instances, prior to affixing the mesh, the mesh is, initially, held in position by pressing on the mesh from outside the body while observing the mesh through a laparoscope or, conversely, pressing upward against the mesh with the use of one or more suitable devices, e.g., an atraumatic grasper or the like. Thereafter, the surgical mesh is often affixed, i.e., sutured or tacked using a fastener, to the cavity wall by conventional suturing techniques. Unfortunately, this method has shortcomings. First, due to movement of the mesh, correctly conforming the mesh to tissue is difficult. Further, correction of mispositioned mesh is difficult and time consuming. There is a need for a device that both positions mesh and fastens mesh to tissue.

### SUMMARY

The present disclosure provides a radio frequency-based surgical implant fixation apparatus. The radio frequency-based surgical implant fixation apparatus includes a housing and a shaft that is operably coupled to housing. A longitudinal axis is defined through the shaft. The housing is adapted to connect to a source of electrosurgical energy. An approximator assembly has an elongated rod that is coaxially coupled to the shaft and is configured to reciprocate therethrough from an extended position to a retracted position. A plurality of delivery arms is operably coupled to a distal end of the elongated rod. The delivery arms are configured to releasably receive a portion of a surgical implant and selectively connect to the energy source to transmit electrosurgical energy to the surgical implant to fuse the surgical implant to tissue upon actuation thereof.

The present disclosure provides a radio frequency-based surgical implant fixation apparatus. The radio frequency-based surgical implant fixation apparatus includes a housing and a shaft that is operably coupled to housing. A longitudinal axis is defined through the shaft. The housing is adapted to connect to a source of electrosurgical energy. A handle assembly includes a movable handle that is movable through an approximation stroke. An approximator assembly has an elongated rod that is coaxially coupled to the shaft and configured to reciprocate therethrough from a retracted position to an extended position through approximation of the movable handle. The elongated rod includes a plurality of compressible and extensible delivery arms operably coupled to a distal end thereof. The plurality of delivery arms is configured to releasably receive a peripheral portion of a surgical implant and selectively connect to the energy source to transmit electrosurgical energy to the surgical implant to fuse the surgical implant to tissue upon actuation thereof. Additional embodiments augment or replace the RF fusion. Another embodiment incorporates a balloon or expansible device that deploys to further control the implant position and press the implant against the tissue.

The present disclosure also provides a method for attaching a surgical implant to tissue. The method includes deploying a surgical implant from a radio frequency-based surgical implant fixation apparatus for positioning the surgical implant adjacent tissue of interest. The surgical implant is, subsequently, expanded so that the surgical implant is substantially taut. The surgical implant is manipulated to force the surgical implant against tissue of interest. And, electrosurgical energy is provided to a portion of the surgical implant to fuse the surgical implant to tissue.

### BRIEF DESCRIPTION OF THE DRAWINGS

Various embodiments of the present disclosure are described hereinbelow with references to the drawings, wherein:
Fig. 1 is a perspective view of a radio frequency-based surgical implant fixation apparatus with an approximator assembly according to an embodiment of the present disclosure;
Fig. 2 is a partial, side view of an elongated rod of the approximator assembly depicted in Fig. 1 in an extended configuration;
Fig. 3A is an enlarged, side view of the area of detail depicted in Fig. 1;
Fig. 3B is a cross-sectional view taken along line-segment 3B-3B in Fig. 3A;
Fig. 4 is a partial, cross-sectional side view of a delivery arm depicted in Fig. 1 having a surgical implant releasably secured thereto and positioned within tissue; and
Fig. 5 is a partial, side view of the approximator assembly according to another embodiment of the present disclosure.

### DETAILED DESCRIPTION

Detailed embodiments of the present disclosure are disclosed herein; however, the disclosed embodiments are merely examples of the disclosure, which may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure.

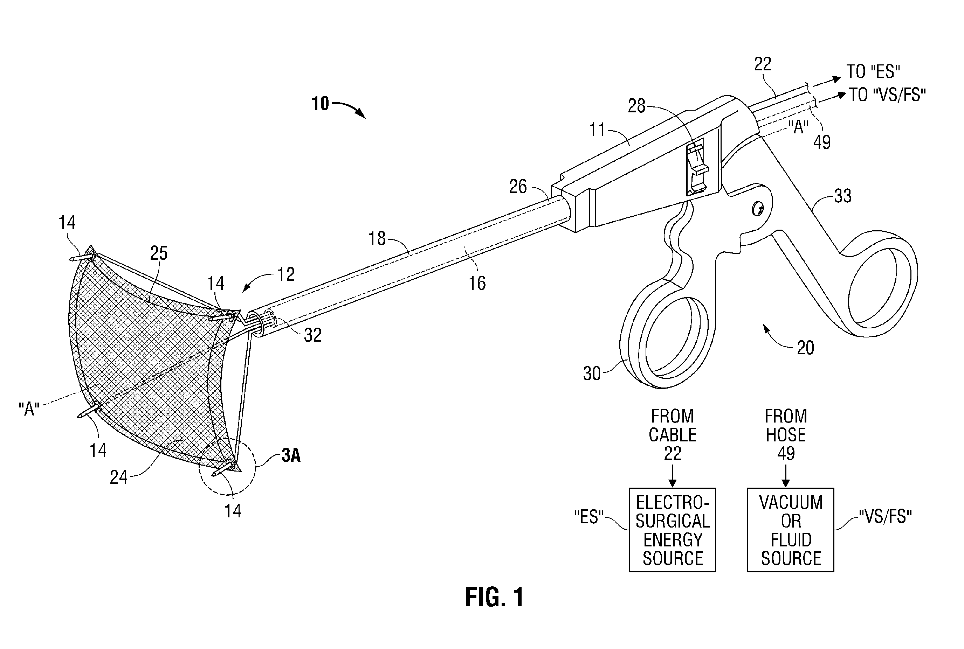

Referring to Fig. 1, a radio frequency-based mesh fixation apparatus 10 (fixation apparatus 10) according to an embodiment of the present disclosure is shown. Fixation apparatus 10 includes an approximator assembly 12 having a plurality of delivery arms 14 positioned at a distal end 32 of an elongated rod 16, a shaft 18, a housing 11, a handle assembly 20, a coaxial cable 22 and a surgical implant 24 releasably coupled to the delivery arms 14, see Fig. 1.

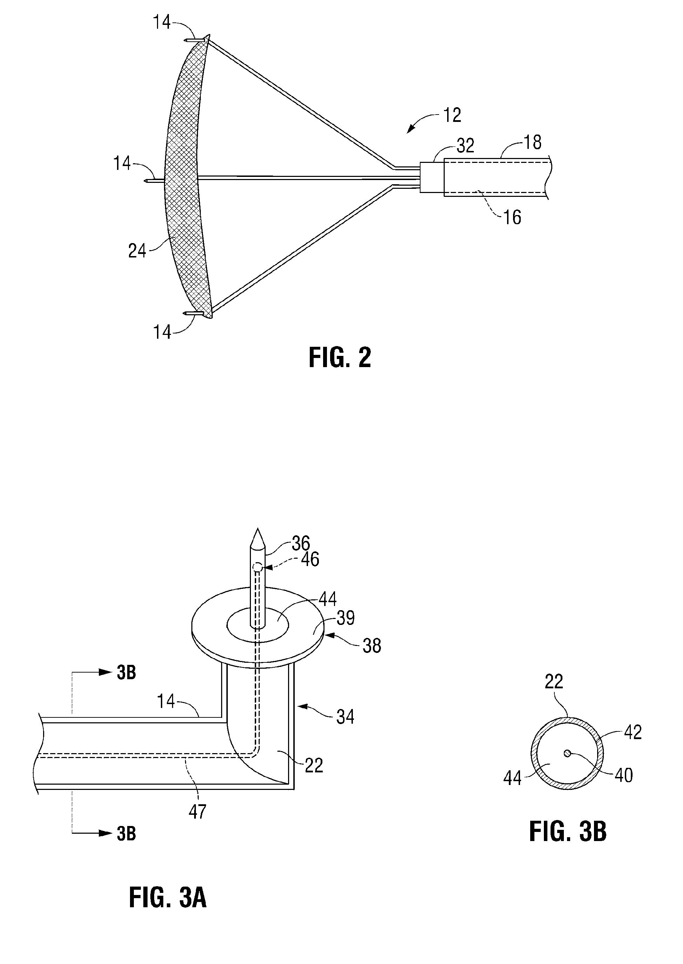

With continued reference to Fig. 1, housing 11 is configured to support one or more components, e.g., a rotating assembly 28, coaxial cable 22, handle assembly 20 and shaft 18, of the fixation apparatus 10 thereon. A proximal end of the housing 11 operably couples to coaxial cable 22 including inner conductor 40, outer conductor 42 and insulative material 44 (see, for example, Fig. 3B) via one or more suitable coupling methods, e.g., a press fit, hub connection, etc. The coaxial cable 22 couples to a suitable source of electrosurgical energy "ES." A distal end of the housing 11 is configured to operably couple to and support the shaft 18.

Shaft 18 extends from the housing 11 and includes a generally elongated configuration. A longitudinal axis "A-A" is defined through the shaft 18. Shaft 18 is configured to house the elongated rod 16 for reciprocation therein from a retracted position (not shown) to an extended position (see Figs. 1 and 2). Shaft 18 includes a proximal end 26 that operably couples to the housing 11 via suitable coupling methods. Shaft 18 is generally in the form of a tubular structure, the width and length of which depends on the type of surgery being performed, the accessibility of the surgical site, the dimensions of the surgical implant 24 and the size of the delivery arms 14 being utilized. For endoscopic or laparoscopic surgical procedures, the shaft 18 is configured to allow insertion thereof into a surgical access port, e.g., a trocar assembly, cannula, SILS™ port or other suitable device.

Handle assembly 20 is operably supported on the housing 11. Handle assembly 20 includes a movable handle 30 (see Fig. 1) that operably connects to the elongated rod 16 for effecting relative reciprocal coaxial movement of the elongated rod 16 between the retracted and extended positions with respect to the shaft 18. Movable handle 30 pivotably couples to a non-movable handle 33 (see Fig. 1) that is operable as a gripping device for a user.

Rotating assembly 28 (see Fig. 1) is operable to rotate the elongated rod 16 including the delivery arms 14 about the longitudinal axis "A-A" relative to the shaft 18 to facilitate positioning the surgical implant 24 adjacent to tissue.

Elongated rod 16 is coaxially-coupled (see Fig. 1 in combination with Fig. 2) to the shaft 18 and is configured to reciprocate therethrough from an extended position to a retracted position. Elongated rod 16 may be made from any suitable material including but not limited to metal, plastic, ceramic and the like. In the illustrated embodiment, elongated rod 16 is made from a relatively resilient plastic material. In certain embodiments, the elongated rod 16 or portion thereof may be configured to articulate, via suitable methods, about the longitudinal axis "A-A" defined through the shaft 18. To effect articulation of the elongated rod 16, and in one particular embodiment, the distal end 32 of the elongated rod 16 may be made from a shape memory alloy, such as, for example, nickel titanium (commonly referred to in the art as "nitinol"). In this instance, the distal end 32 is configured to transform from an unarticulated configuration, i.e., an austenite phase, to an articulated configuration, i.e., a martensite phase. Alternatively, the elongated rod 16 may be configured to articulate via mechanical methods, e.g., pulleys, cables, links, etc. Distal end 32 of the elongated rod 16 is configured to operably support a plurality of delivery arms 14.

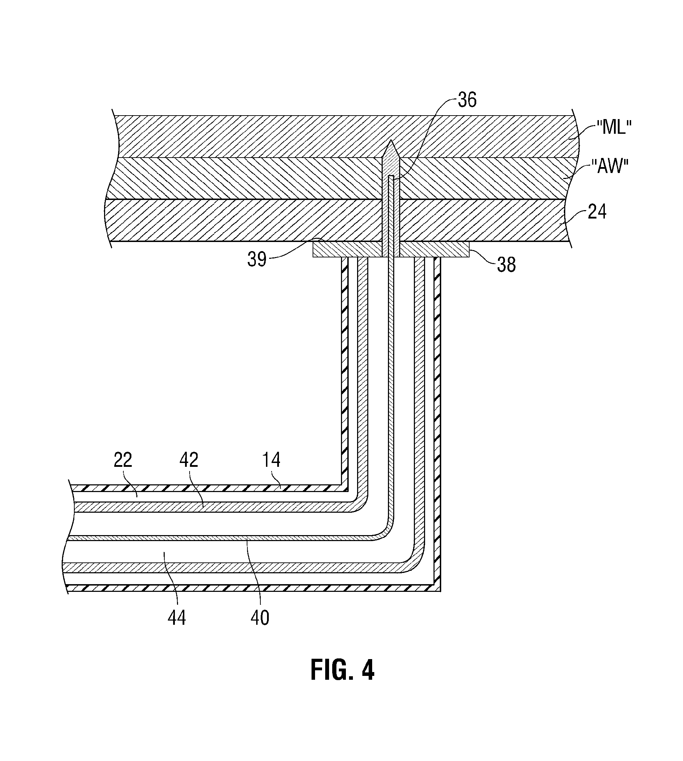

Delivery arms 14 (four (4) delivery arms are shown in the representative drawings) are positioned and operably secured at the distal end 32 of an elongated rod 16 via one or more suitable securementg methods, e.g., soldering, brazing, welding, press or friction fit, etc. In the illustrated embodiment, the delivery arms 14 are configured to releasably receive a peripheral portion of surgical implant 24 to position the surgical implant 24 adjacent tissue, e.g., a surgical opening or wound in an abdominal wall "AW", and for pressing the surgical implant 24 thereagainst. To facilitate positioning the surgical implant 24 adjacent to tissue (e.g., abdominal wall "AW", see Fig. 4) or pressing the surgical implant thereagainst, each delivery arm 14 includes a generally bent or slanted configuration adjacent and proximal to an electrically conductive distal end 34. The slanted or bent configuration of the delivery arms 14 allows an end user to obtain a mechanical advantage within the relatively small area or confines of the body cavity.

Each delivery arm of the delivery arms 14 is independently movable with respect to the other delivery arms to allow proper apposition of the surgical implant 24 against tissue. Secondary instruments, such as atraumatic graspers (and the like) may be utilized to effect movement of each delivery arm relative to the plurality of delivery arms 14. Alternatively, one or more steering mechanisms or control mechanisms may be in operable communication with the delivery arms 14 and/or the elongated rod 16 to effect independent movement or control of each delivery.

Delivery arms 14 are fabricated from a resilient material that allow each delivery arm 14 to transition from a compressed state when the elongated rod 16 is in the retracted position (not explicitly shown), to an expanded state when the elongated rod 16 is in the extended position (see Figs. 1 and 2). In embodiments, delivery arms 14 may be fabricated from silicone, polyvinyl resins, polyethylene, resilient polyacetals, polyurethane, resilient polycetals, polyurethane, synthetic rubbers, Teflon, tetrafluorethylene fluorocarbon polymer, spring-tempered steel and spring tempered stainless steel, shape memory alloy or other suitable material that allow the delivery arms 14 to move or transition from the compressed state to the expanded state. Alternatively, a supplemental device, mechanism or structure may operably couple to the approximator assembly 12 and/or the delivery arms 14 to facilitate moving the delivery arms 14 from the compressed state to the expanded state. For example, one or more wires, cables or pulleys may operably couple to the delivery arms 14 to move the delivery arms 14 from the compressed state to the expanded state and vice versa.

With reference to Figs. 3A and 3B, the delivery arms 14 are configured to transmit electrosurgical energy at a desired frequency and duration to the surgical implant 24 to fuse the surgical implant 24 to tissue. As can be appreciated, the frequency and duration of the electrosurgical energy that is transmitted to the delivery arms 14 may be varied to accommodate a specific type of surgical implant 24 (or material that the surgical implant 24 is made from), a specific surgical procedure, a specific type of tissue that the surgical implant 24 is configured to secure to, etc. Each delivery arm of the delivery arms 14 includes a respective electrically conductive distal end 34 that has an active electrode 36 in electrical communication with the inner conductor 40 and a return electrode 38 in electrical communication with an outer conductor 44, see Figs. 3A and 3B. In the illustrated embodiment, the active electrode 36 and return electrode 38 are energizable in a bipolar configuration. Alternatively, a monopolar electrode configuration may be utilized. In this instance, a return pad (not shown) may be utilized to function as a return electrode 38. The RF energy may be applied by known methods and may have a frequency from DC to hundreds of KHZ. A typical frequency is 472 KHZ.

Continuing with reference to Fig. 3A, active electrode 36 includes a relatively pointed or sharp tip configuration operable to penetrate tissue and configured to facilitate secure placement of the surgical implant 24 against tissue during electrical activation. That is, the pointed tip of the active electrode 36 functions as a temporary anchoring device that maintains the surgical implant 24 in a relatively fixed position with respect to the tissue covered thereby, i.e., eliminates undesirable movement of the surgical implant 24 when the surgical implant 24 is being fused to tissue.

Return electrode 38 includes a generally flat circumferential base 39 operable to releasably support the surgical implant 24 thereon (see Figs. 3A and 4). The flat base 39 provides a relatively even distribution of the transmitted electrosurgical energy to the surgical implant 24 to achieve a consistent and uniform fusion or union between the surgical implant 24 and tissue.

In certain instances, it may prove advantageous to provide an exterior surface of the active electrode 36 and/or return electrode 38 with one or more lubricious materials thereon, e.g., a coating of polytetrafluoroethylene (PTFE), to decrease charring and/or sticking of the surgical implant 24 to the active and/or return electrodes 36 and 38, respectively. A layer of insulative material 44 is operably disposed between the active and return electrodes 36 and 38, see Figs. 3A and 3B.

With reference again to Figs. 1 and 2, surgical implant 24 may be made from any suitable type of material including, but not limited to, water-repellant, breathable laminated fabric (such as the type sold under the registered trademark GORETEX^{®}), plastic, and/or bio-absorbable materials. Such bio-absorbable materials include synthetic polymers but more preferably include collagen materials derived from human and animal sources, such as acellular collagen matrix. For example, one material is porcine dermis, rendered acellular and inert via organic and enzymatic extraction processes that is then cross-linked with non-calcifying HMDI to make it durable and highly resistant to breakdown by naturally occurring collagenases for enhanced durability in complex repairs (such as the bio-absorbable material sold under the registered trademark PERMACOL^{®}). In the illustrated embodiment, the surgical implant 24 is made from PERMACOL^{®}. The surgical implant 24 may be configured in a variety of shapes or forms. For example, the surgical implant 24 may be an implantable sheet material that may be flat, ball-shaped, cylindrically or tubularly rolled, as well as any other configuration within the knowledge of those skilled in the art. In addition, the surgical implant 24 may be a mesh-like sheet, shown in Figs. 1 and 2 or a solid sheet (not shown). For illustrative purposes, the surgical implant 24 is disclosed herein as a mesh material that may include one or more varieties of weave configurations. In certain instances, the surgical implant 24 may be a combination of a solid sheet and a mesh sheet. For example, the surgical implant 24 may include a solid inner configuration and a mesh outer configuration. Alternatively, the surgical implant 24 may have a mesh inner configuration that is tightly woven and a mesh outer configuration that is loosely woven. While the surgical implant 24 may be discussed as a surgical mesh, the surgical implant 24 embodies a wide variety of configurations. Further, the use of the term "surgical mesh" or "mesh" is not intended to limit the types of surgical implants that may be used in the present invention.

Surgical implant 24 is configured such that electrosurgical energy transmitted to the electrodes 36, 38 fuses the surgical implant 24 (or a portion thereof) to tissue positioned thereagainst. The surgical implant 24 may be configured such that the entirety of the surgical implant 24 fuses to tissue during the transmission of electrosurgical energy to the electrodes 36, 38, or only a portion of the surgical implant 24 fuses to tissue.

In certain embodiments, a peripheral portion 25 of the surgical implant 24 adjacent the electrodes may be made from or coated with metal, e.g., silver, nickel, titanium, gold, etc., that is configured to augment tissue fusion. In particular, the metal around the peripheral portion 25 is configured to provide an even distribution of electrosurgical energy to the surgical implant 24 to help fuse the surgical implant 24 to tissue and provide a more uniform and consistent fusion or union therebetween. Metal around the peripheral portion 25 can also assist by creating and distributing heat to the mesh and tissue. When creating heat in a metalized mesh, the energy applied by the electrode may need to be at a higher frequency, for example 13.56 MHZ.

Operation of the fixation device 10 is described in terms of a method for attaching a surgical implant 24 to tissue to repair a ventral hernia.

In use, shaft 18 is inserted through an access port previously affixed to an opening in tissue, i.e., though a muscle layer "ML" (see Fig. 4), for positioning the shaft 18 adjacent tissue of interest.

Elongated rod 16 is, initially, in a retracted position with the delivery arms 14 in a compressed state. Proximal movement of the movable handle 30 moves the rod 16 from the retracted position to the extended position (see Figs. 1 and 2). In the extended position, delivery arms 14 transition from the compressed state to the expanded state, which, in turn, moves the surgical implant 24 from the compressed state to the expanded state (see Figs. 1 and 2). In the expanded state, the surgical implant 24 is substantially taut, see Fig. 2. Subsequently, the delivery arms 14 are utilized to position the surgical implant 24 adjacent the object tissue and force is applied to press the surgical implant 24 against the tissue (see Fig. 4).

Thereafter, electrosurgical energy is transmitted from electrosurgical energy source "ES" to the active electrodes 36 and return electrodes 38 of the delivery arms 14 and, thus, to a peripheral edge of the surgical implant 24 to fuse the surgical implant 24 to tissue (see Fig. 4).

As can be appreciated, the above-mentioned shortcomings typically associated with conventional hernia repair methods are reduced and/or eliminated. That is, the surgical implant 24 is maintained in a substantially fixed orientation while the surgical implant 24 is being fused to the abdominal wall "AW" and, thus, the likelihood of the surgical implant 24 moving during attachment is reduced, if not eliminated.

From the foregoing and with reference to the various figure drawings, those skilled in the art will appreciate that certain modifications can also be made to the present disclosure without departing from the scope of the same. For example, to facilitate positioning the surgical implant 24 adjacent tissue, one or more apertures 46 (shown in phantom in Fig. 3A for illustrative purposes) may be defined in one or more of the delivery arms 14. In particular, aperture 46 may be operably disposed on the active electrode 36 (or on the return electrode 38) and in fluid communication with a suction or vacuum source "VS" that is configured to evacuate air between the surgical implant 24 and tissue. One or more conduits or lumens 47 (also shown in phantom in Fig. 3A) may provide fluid communication between the aperture 46 and the vacuum source. In this instance, the lumen 47 extends from the aperture 46 through a corresponding delivery arm 14 and the elongated rod 16 and connects to the vacuum source "VS" via a hose 49 or other suitable device. As can be appreciated, as air is evacuated from between the surgical implant 24 and tissue, the surgical implant 24 is "pulled" or "drawn" thereagainst; this facilitates fusing the surgical implant 24 to tissue, i.e., a better fusion between the surgical implant 24 and tissue may be achieved.

To facilitate pulling tissue and surgical implant 24 toward one another or maintaining the surgical implant 24 against tissue, the return electrode 38 may include a generally "cup-like" configuration, i.e., the return electrode 38 may function as a suction cup. In this instance, a peripheral edge of the return electrode 38 may be curved or otherwise configured to provide suction around a perimeter of the return electrode 38.

As can be appreciated, plumes of smoke may develop when the surgical implant 24 is being fused to tissue. Accordingly, and in certain embodiments, the aperture 46 may be operable to evacuate plumes of smoke from the surgical site to provide better visualization of the surgical site to the surgeon.

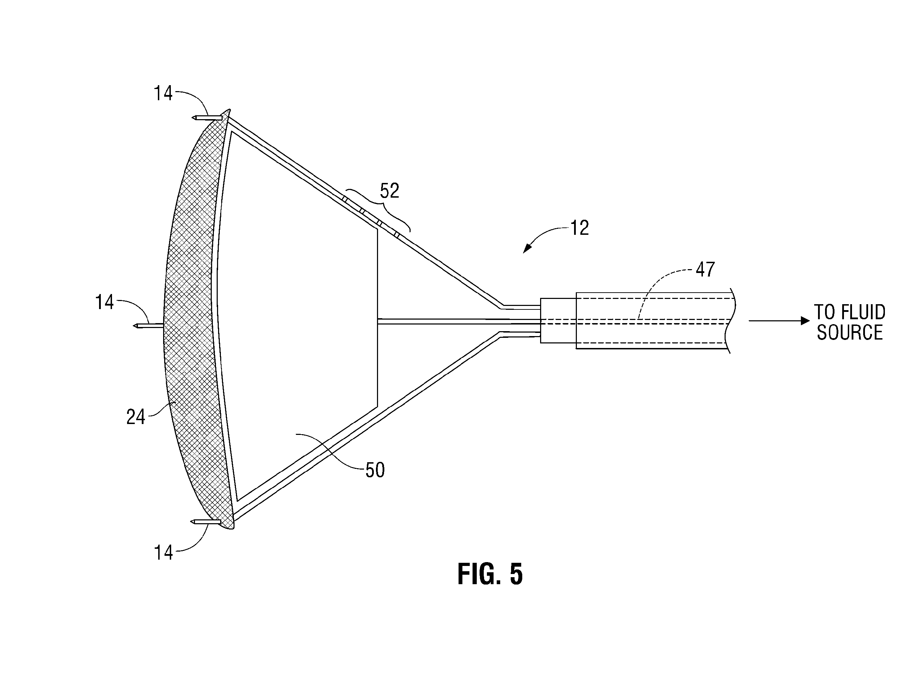

A supplemental device may be utilized to facilitate pressing the surgical implant 24 against tissue prior to or during the application of electrosurgical energy. In particular, in some embodiments, the approximator assembly 12 may include or be in operative communication with an expandable member, such as, for example, a balloon 50 (see Fig. 5). In this particular embodiment, balloon 50 may be operably coupled to the elongated rod 16 and configured to press the surgical implant 24 against tissue when the balloon 50 is in an expanded state and surgical implant 24 is being fused to tissue. In this instance, the balloon 50 may be in fluid communication with one or more fluid sources, i.e., the vacuum source may also be configured to provide one or more suitable fluids, e.g., saline, air, water, etc., to the balloon 50. In certain instances, as in the embodiment illustrated in Fig. 5, the temperature of the fluid may be regulated (e.g., the fluid may be chilled or heated) to facilitate maintaining the surgical implant 24 (or portion thereof) at a predetermined threshold temperature.

In certain embodiments, one or more sensors 52 (see Fig. 5) may be operably associated with the approximator assembly 12 to provide feedback and/or data pertaining to the fixation device 10 including the fixation arms 14, surgical implant 24 and/or the surgical site. In particular, sensors 52 may be operably coupled to one or more of the fixation arms 14 (see Fig. 5). The sensors 52 may, for example, be configured to detect if the fixation arms 14 are deployed, if the surgical implant 24 is pressed against tissue, or if temperature regulation is "offline" or if a predetermined temperature has been reached at the surgical implant, tissue and/or active and return electrodes 36 and 38, respectively. In this instance, the sensors 52 may be in operative communication with a microprocessor (not shown) of the electrosurgical energy source "ES" to process the relevant feedback and/or data supplied thereto.

Balloon 50 may be thermally or electrically conductive or non-conductive to achieve a desired surgical effect. For example, an exterior surface (or portion thereof) of the balloon 50 may include one or more conductive materials disposed thereon or may be made of a conductive material. The conductive exterior surface of the balloon 50 may be configured to facilitate or direct current flow from the active electrode 36 to the return electrode 38 when the surgical implant 24 is being fused to tissue.

Additional methods of attaching the mesh to tissue can also be to replace or augment using fusion at the end of arms 14. Mechanical methods include the use of known tacks and fasteners for hernia fixation. For instance, electrode 36 may be replaced with detachable barbed fasteners. Biological glues or bioadhesives that can also provide adhesion between a living biological tissue and a synthetic or biological material (e.g., hernia patch/mesh); thus, providing an attachment between tissue and the patch/mesh. The glues can be either multi component (e.g., fibrin glue or fibrin sealant), single component (e.g., cyanoacrylate) that are chemically or thermally initiated, UV initiated by light guides in arms 14 or any other glue suitable for clinical use. The glue may be dispensed automatically at the ends of the arms 14 or preapplied to the mesh.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A radio frequency-based surgical implant fixation apparatus, comprising:
   a housing having a shaft operably coupled thereto and defining a longitudinal axis therethrough, the housing adapted to connect to a source of electrosurgical energy; and
   an approximator assembly having an elongated rod coaxially coupled to the shaft and configured to reciprocate therethrough from a retracted position to an extended position, the elongated rod including a plurality of delivery arms operably coupled to a distal end thereof, the plurality of delivery arms configured to releasably receive a portion of a surgical implant and connect to the energy source to selectively transmit electrosurgical energy to at least a portion of the surgical implant to fuse the at least a portion of the surgical implant to tissue upon actuation thereof.
2. A radio frequency-based surgical implant fixation apparatus according to paragraph 1, wherein each delivery arm operably connects to a coaxial cable having an inner conductor, an outer conductor and an insulative material therebetween.
3. A radio frequency-based surgical implant fixation apparatus according to paragraph 2, wherein each delivery arm includes a respective electrically conductive distal end having an active electrode in electrical communication with the inner conductor of the coaxial cable and a return electrode in electrical communication with the outer conductor of the coaxial cable.
4. A radio frequency-based surgical implant fixation apparatus according to paragraph 3, wherein at least one of the active electrodes includes a relatively pointed tip operable to penetrate tissue and the return electrode includes a generally flat circumferential base operable to releasably support the surgical implant thereon and the insulative material is operably disposed between the active electrode and the return electrode.
5. A radio frequency-based surgical implant fixation apparatus according to paragraph 3, wherein at least one aperture is defined in one of the active electrode and return electrode.
6. A radio frequency-based surgical implant fixation apparatus according to paragraph 5, wherein the at least one aperture is in fluid communication with a suction source configured to evacuate air from between the surgical implant and tissue to facilitate positioning the surgical implant thereagainst in a substantially taut configuration.
7. A radio frequency-based surgical implant fixation apparatus according to paragraph 6, wherein the at least one aperture is operable to evacuate plumes of smoke caused by the fusing of the surgical implant to tissue.
8. A radio frequency-based surgical implant fixation apparatus according to paragraph 1, wherein each delivery arm is fabricated from a resilient material that allows each delivery arm to transition from a compressed state and to an expanded state when the elongated rod is moved from the retracted position to the extended position.
9. A radio frequency-based surgical implant fixation apparatus according to paragraph 8, wherein each delivery arm includes a bent configuration adjacent the electrically conductive distal end.
10. A radio frequency-based surgical implant fixation apparatus according to paragraph 8, wherein the resilient material is selected from the group consisting of silicone, polyvinyl resins, polyethylene, resilient polyacetals, polyurethane, resilient polycetals, polyurethane, synthetic rubbers, teflon, tetrafluorethylene fluorocarbon polymer, spring-tempered steel, spring tempered stainless steel and shape-memory alloy.
11. A radio frequency-based surgical implant fixation apparatus according to paragraph 1, wherein the surgical implant is a surgical mesh.
12. A radio frequency-based surgical implant fixation apparatus according to paragraph 11, wherein a peripheral portion of the surgical mesh is made from a conductive metal that is configured to enhance electrosurgical energy transfer to tissue to augment tissue fusion.
13. A radio frequency-based surgical implant fixation apparatus according to paragraph 12, wherein the metal is selected from the group consisting of silver, nickel, titanium and gold.
14. A radio frequency-based surgical implant fixation apparatus according to paragraph 1, further comprising:
   a handle assembly having a movable handle operably connected to the elongated rod for effecting relative reciprocal coaxial movement of the elongated rod between the retracted and extended position with respect to the shaft and a non-movable handle operable as a gripping device for a user.
15. A radio frequency-based surgical implant fixation apparatus according to paragraph 1, further comprising:
   a rotating device for rotating the elongated rod including the delivery arms about the longitudinal axis relative to the shaft.
16. A radio frequency-based surgical implant fixation apparatus according to paragraph 1, further comprising:
   a balloon configured to apply pressure to said implant when the elongated rod is in the extended position.
17. A radio frequency-based surgical implant fixation apparatus, comprising:
   a housing having a shaft operably coupled thereto and defining a longitudinal axis therethrough, the housing adapted to connect to a source of electrosurgical energy;
   a handle assembly including a movable handle; and
   an approximator assembly having an elongated rod coaxially coupled to the shaft and configured to reciprocate therethrough from a retracted position to an extended position through approximation of the movable handle, the elongated rod including a plurality of resilient delivery arms operably coupled to a distal end thereof, the plurality of delivery arms configured to releasably receive a peripheral portion of a surgical implant and to selectively connect to the energy source to transmit electrosurgical energy to at least a portion of the surgical implant to fuse the at least a portion of the surgical implant to tissue upon actuation thereof.
18. A method for attaching a surgical implant to tissue, comprising:
   deploying a surgical implant from a radio frequency-based surgical implant fixation apparatus for positioning the surgical implant adjacent tissue of interest;
   expanding the surgical implant so that the surgical implant is substantially taut;
   manipulating the surgical fixation apparatus to force the surgical implant against tissue of interest; and
   providing electrosurgical energy to at least a portion of the surgical implant to fuse the surgical implant to tissue.
19. A method according to paragraph 17, wherein the step of manipulating includes piercing tissue with a distal end of at least one delivery arm of the radio frequency-based surgical implant fixation apparatus to facilitate fusing the surgical implant to tissue.
20. A method according to paragraph 18, wherein the step of providing electrosurgical energy to the at least a portion of the surgical implant is achieved via an active electrode and a return electrode operably disposed on a distal end of each delivery arm.
21. A method according to paragraph 18, wherein the step of expanding the surgical implant includes the step of evacuating air from between the surgical implant and tissue to facilitate positioning the surgical implant against the tissue in the substantially taut configuration.
22. A method according to paragraph 18, wherein the step of expanding the surgical implant includes the deploying a balloon.
23. A method according to paragraph 18, wherein the step of manipulating the surgical fixation apparatus to force the surgical implant against tissue of interest uses a balloon.
   1a. A radio frequency-based surgical implant fixation apparatus, comprising:
      a housing having a shaft operably coupled thereto and defining a longitudinal axis therethrough, the housing adapted to connect to a source of electrosurgical energy; and
      an approximator assembly having an elongated rod coaxially coupled to the shaft and configured to reciprocate therethrough from a retracted position to an extended position, the elongated rod including a plurality of delivery arms operably coupled to a distal end thereof, the plurality of delivery arms configured to releasably receive a portion of a surgical implant and connect to the energy source to selectively transmit electrosurgical energy to at least a portion of the surgical implant to fuse the at least a portion of the surgical implant to tissue upon actuation thereof.
   2a. A radio frequency-based surgical implant fixation apparatus according to paragraph 1a, wherein each delivery arm operably connects to a coaxial cable having an inner conductor, an outer conductor and an insulative material therebetween.
   3a. A radio frequency-based surgical implant fixation apparatus according to paragraph 1a or paragraph 2a, wherein each delivery arm includes a respective electrically conductive distal end having an active electrode in electrical communication with the inner conductor of the coaxial cable and a return electrode in electrical communication with the outer conductor of the coaxial cable.
   4a. A radio frequency-based surgical implant fixation apparatus according to paragraph 3a, wherein at least one of the active electrodes includes a relatively pointed tip operable to penetrate tissue and the return electrode includes a generally flat circumferential base operable to releasably support the surgical implant thereon and the insulative material is operably disposed between the active electrode and the return electrode.
   5a. A radio frequency-based surgical implant fixation apparatus according to paragraph 3a or paragraph 4a, wherein at least one aperture is defined in one of the active electrode and return electrode.
   6a. A radio frequency-based surgical implant fixation apparatus according to paragraph 5a, wherein the at least one aperture is in fluid communication with a suction source configured to evacuate air from between the surgical implant and tissue to facilitate positioning the surgical implant thereagainst in a substantially taut configuration.
   7a. A radio frequency-based surgical implant fixation apparatus according to paragraph 5a or paragraph 6a, wherein the at least one aperture is operable to evacuate plumes of smoke caused by the fusing of the surgical implant to tissue.
   8a. A radio frequency-based surgical implant fixation apparatus according to any of paragraphs 1a to 7a, wherein each delivery arm is fabricated from a resilient material that allows each delivery arm to transition from a compressed state and to an expanded state when the elongated rod is moved from the retracted position to the extended position.
   9a. A radio frequency-based surgical implant fixation apparatus according to any of paragraphs 1a to 8a, wherein each delivery arm includes a bent configuration adjacent the electrically conductive distal end.
   10a. A radio frequency-based surgical implant fixation apparatus according to paragraph 8a, wherein the resilient material is selected from the group consisting of silicone, polyvinyl resins, polyethylene, resilient polyacetals, polyurethane, resilient polycetals, polyurethane, synthetic rubbers, teflon, tetrafluorethylene fluorocarbon polymer, spring-tempered steel, spring tempered stainless steel and shape-memory alloy.
   11a. A radio frequency-based surgical implant fixation apparatus according to any of paragraphs 1a to 10a, wherein the surgical implant is a surgical mesh.
   12a. A radio frequency-based surgical implant fixation apparatus according to paragraph 11a, wherein a peripheral portion of the surgical mesh is made from a conductive metal that is configured to enhance electrosurgical energy transfer to tissue to augment tissue fusion.
   13a. A radio frequency-based surgical implant fixation apparatus according to paragraph 12a wherein the metal is selected from the group consisting of silver, nickel, titanium and gold.
   14a. A radio frequency-based surgical implant fixation apparatus according to any of paragraphs 1a to 13a, further comprising:
      a handle assembly having a movable handle operably connected to the elongated rod for effecting relative reciprocal coaxial movement of the elongated rod between the retracted and extended position with respect to the shaft and a non-movable handle operable as a gripping device for a user; and/or further comprising a rotating device for rotating the elongated rod including the delivery arms about the longitudinal axis relative to the shaft; and/or further comprising a balloon configured to apply pressure to said implant when the elongated rod is in the extended position.
   15a. A radio frequency-based surgical implant fixation apparatus according to paragraph 1a, comprising:
      a housing having a shaft operably coupled thereto and defining a longitudinal axis therethrough, the housing adapted to connect to a source of electrosurgical energy;
      a handle assembly including a movable handle; and
      an approximator assembly having an elongated rod coaxially coupled to the shaft and configured to reciprocate therethrough from a retracted position to an extended position through approximation of the movable handle, the elongated rod including a plurality of resilient delivery arms operably coupled to a distal end thereof, the plurality of delivery arms configured to releasably receive a peripheral portion of a surgical implant and to selectively connect to the energy source to transmit electrosurgical energy to at least a portion of the surgical implant to fuse the at least a portion of the surgical implant to tissue upon actuation thereof.

## Claims

1. A radio frequency-based surgical implant fixation apparatus, comprising:
a housing having a shaft operably coupled thereto and defining a longitudinal axis therethrough, the housing adapted to connect to a source of electrosurgical energy; and
an approximator assembly having an elongated rod coaxially coupled to the shaft and configured to reciprocate therethrough from a retracted position to an extended position, the elongated rod including a plurality of delivery arms operably coupled to a distal end thereof, the plurality of delivery arms configured to releasably receive a portion of a surgical implant and connect to the energy source to selectively transmit electrosurgical energy to the portion of the surgical implant to fuse the portion of the surgical implant to tissue upon actuation thereof, wherein at least one aperture is defined in the plurality of delivery arms, the at least one aperture being in fluid communication with a suction source configured to evacuate air from between the surgical implant and tissue to facilitate positioning the surgical implant thereagainst in a substantially taut configuration.

2. A radio frequency-based surgical implant fixation apparatus according to claim 1, wherein each delivery arm operably connects to a coaxial cable having an inner conductor, an outer conductor and an insulative material therebetween, each delivery arm including a respective electrically conductive distal end having an active electrode in electrical communication with the inner conductor of the coaxial cable and a return electrode in electrical communication with the outer conductor of the coaxial cable.

3. A radio frequency-based surgical implant fixation apparatus according to claim 2, wherein at least one of the active electrodes includes a relatively pointed tip operable to penetrate tissue and the return electrode includes a generally flat circumferential base operable to releasably support the surgical implant thereon and the insulative material is operably disposed between the active electrode and the return electrode.

4. A radio frequency-based surgical implant fixation apparatus according to claim 3, wherein the at least one aperture is operable to evacuate smoke caused by the fusing of the surgical implant to tissue.

5. A radio frequency-based surgical implant fixation apparatus according to claim 1, wherein each delivery arm is fabricated from a resilient material that allows each delivery arm to transition from a compressed state and to an expanded state when the elongated rod is moved from the retracted position to the extended position.

6. A radio frequency-based surgical implant fixation apparatus according to claim 2, wherein each delivery arm includes a bent configuration adjacent the electrically conductive distal end.

7. A radio frequency-based surgical implant fixation apparatus according to claim 5, wherein the resilient material is selected from the group consisting of silicone, polyvinyl resins, polyethylene, resilient polyacetals, polyurethane, resilient polycetals, polyurethane, synthetic rubbers, teflon, tetrafluorethylene fluorocarbon polymer, spring-tempered steel, spring tempered stainless steel and shape-memory alloy.

8. A radio frequency-based surgical implant fixation apparatus according to claim 1, wherein the surgical implant is a surgical mesh.

9. A radio frequency-based surgical implant fixation apparatus according to claim 8, wherein a peripheral portion of the surgical mesh is made from a conductive metal that is configured to enhance electrosurgical energy transfer to tissue to augment tissue fusion.

10. A radio frequency-based surgical implant fixation apparatus according to claim 9, wherein the conductive metal is selected from the group consisting of silver, nickel, titanium and gold.

11. A radio frequency-based surgical implant fixation apparatus according to claim 1, further comprising:
a handle assembly having a movable handle operably connected to the elongated rod for effecting relative reciprocal coaxial movement of the elongated rod between the retracted and extended position with respect to the shaft and a non-movable handle operable as a gripping device for a user.

12. A radio frequency-based surgical implant fixation apparatus according to claim 1, further comprising:
a rotating device for rotating the elongated rod including the delivery arms about the longitudinal axis relative to the shaft.

13. A radio frequency-based surgical implant fixation apparatus according to claim 1, further comprising:
a balloon configured to apply pressure to said implant when the elongated rod is in the extended position.
